# EUROPÄISCHE PATENTSCHRIFT

(11) **EP 0 363 610 B1**
(45) Veröffentlichungstag und Bekanntmachung des Hinweises auf die Patenterteilung: **09.03.1994**
(21) Anmeldenummer: 89115404.9
(22) Anmeldetag: 21.08.1989
(51) Int. Cl.: A61B 3/024

(54) **Vorrichtung zum Prüfen visueller Funktionen eines menschlichen Auges**
Device for examining the visual functions of a human eye
Dispositif d'examen de fonctions visuelles d'un oeil humain

(30) Priorität: 22.09.1988 CH 3507/88
(43) Veröffentlichungstag der Anmeldung: 18.04.1990
(73) Patentinhaber: Interzeag AG, CH-8952 Schlieren (CH)
(72) Erfinder: Billeter, Ernst, CH-8952 Bergdietikon (CH); Bebié, Hans, Dr. Prof., CH-3019 Oberbottigen (CH)
(74) Vertreter: Fillinger, Peter, Dr.

(56) Entgegenhaltungen:
- DE-A- 2 843 287
- GB-A- 2 096 791
- US-A- 3 718 386
- US-A- 4 012 128

## Beschreibung

Die vorliegende Erfindung bezieht sich auf eine Vorrichtung zum Prüfen visueller Funktionen eines menschlichen Auges gemäss dem Oberbegriff des Anspruchs 1.

Unter Prüfung visueller Funktionen fallen: Perimetrie, Bestimmung der lokalen Sehschärfe auf der Netzhaut, Bestimmung der Flimmerfrequenz, Bestimmung der Kontrast- oder Farbkontrastempfindlichkeit etc. Derartige Vorrichtungen sind z.B. unter der Bezeichnung "Automatische Perimeter" seit Jahren bekannt. Bei diesen automatischen Perimetern wird das zu untersuchende Auge im Zentrum (oder nahe des Zentrums) einer Halbkugel fixiert. Die Beobachtungsrichtung verläuft zum Apex der Cupolaoberfläche. Die Stimuli werden von einem Projektor auf die innenseitige Halbkugelfläche der Cupola projiziert und der Patient hat nach dem Darbieten eines Stimulus seine Antwort (gesehen oder nicht gesehen) zu signalisieren. Diese bekannten Perimeter sind ungewöhnlich gross und entsprechend teuer, wobei die Cupola allein einen Durchmesser von 60 bis 100 cm aufweist. Zieht man in Betracht, dass das Zentrum der Halbkugelfläche auf der Höhe des Auges eines sitzenden Patienten liegt, erreicht die ganze Einrichtung notwendigerweise eine Höhe von bis zu zwei Metern.

Diese bekannten Perimeter sind zwar vorzügliche Untersuchungsgeräte, doch eignen sie sich vorwiegend nur für Spitäler und ophtalmologische Praxen, in denen genügend Raum verfügbar ist. Für den praktizierenden Augenarzt oder Optiker sind sie oft zu raumbeanspruchend und/oder zu teuer.

Zur Verkleinerung solcher Geräte wird in der GB-A 20 96 791 vorgeschlagen, den Radius der Cupola auf ca. 10cm zu reduzieren und die Cupola wie eine Maske auf das Patientengesicht aufzusetzen. Da die kleinste Scharfsichdistanz eines normalsichtigen Auges mehr als 25cm beträgt, hat diese ältere Lösung den Nachteil, dass das Patientenauge die dargebotenen Stimuli nur verschwommen wahrnimmt, was zu Fehlmessungen führen kann.

Die DE-A 28 43 287 bemängelt bei den vorgenannten Geräten die Ungenauigkeit, mit der sich deren Messwerte den während der Untersuchung tatsächlich stimulierten Stellen der Netzhaut zuordnen lassen. Sie schlägt daher Mittel vor, welche mit der Stimulation von Netzhautstellen gleichzeitig den Augenhintergrund photografisch aufzeichnen. Diese Mittel machen indessen das Gerät konstruktiv aufwendig und platzbeanspruchend.

Die vorliegende Erfindung stellt sich daher die Aufgabe, ein Gerät der eingangs erwähnten Art zu miniaturisieren, ohne dass die Qualität seiner Messungen beeinträchtigt wird.

Erfindungsgemäss wird diese Aufgabe gelöst durch die kennzeichnenden Merkmale des Anspruches 1.

Durch die Erfindung entfällt die Cupola und damit der raumbeanspruchendste Teil der bekannten Perimeter.

Durch die Erfindung werden die Stimuli auf kleinster Fläche dargeboten und vom Patienten mittels einer Lupe betrachtet, wodurch eine augenfällige Miniaturisierung der Erfindung erreicht wird.

Die Ausführungsform nach Anspruch 4 ermöglicht es, in einfachster Weise eine Fixationsmarke, die Umfeldbeleuchtung und eine Beobachtung des Patientenauges einzublenden.

Bei den Ausführungsformen nach den Ansprüchen 6, 7 und 8 sind Möglichkeiten gezeigt, nebst den Farben auch die Helligkeit, die Dauer und das Leuchtdichteprofil von Stimulus bzw. Umfeld zu ändern.

Die Ausführungsform nach Anspruch 10 erlaubt es, an jedem für den Stimulus wählbaren Ort des Gesichtsfeldes eine scharfe Abbildung des Stimulus auf der Netzhaut des Beobachterauges zu erzeugen.

Sind entsprechend des Anspruchs 12 die Okularlinsen in Richtung ihrer optischen Achse verstellbar, können sphärische Sehfehler des Patienten korrigiert werden.

Die Ausführungsform nach Anspruch 13 ermöglicht es, wie bei den bekannten Perimetern, dem zu untersuchenden Auge ein Hilfsmittel in Form einer Fixationsmarke anzubieten, die es ihm erleichtert, während der ganzen Untersuchung seines Auges die Blickrichtung beizubehalten. Zudem entfällt das bei bekannten Perimetern hierfür erforderliche Loch im Gesichtsfeld und jene apparativen Teile, die dazu bestimmt sind diesen Mangel zu beheben.

Bei der Ausführungsform nach Anspruch 15 kann das Patientenauge während der ganzen Untersuchung ohne Irritation beleuchtet und vom Operateur auf dem Bildschirm beobachtet werden.

Anhand der beiliegenden schematischen Zeichnung wird die Erfindung beispielsweise erläutert. Es zeigen:
- Fig. 1: eine schematische Darstellung eines automatischen Perimeters,
- Fig. 2: eine detaillierte Darstellung des eigentlichen Perimeters gemäss Fig. 1,
- Fig. 3: eine räumliche Darstellung des Perimeters nach Fig. 2 und
- Fig. 4: ein zweites Ausführungsbeispiel eines eigentlichen Perimeters.

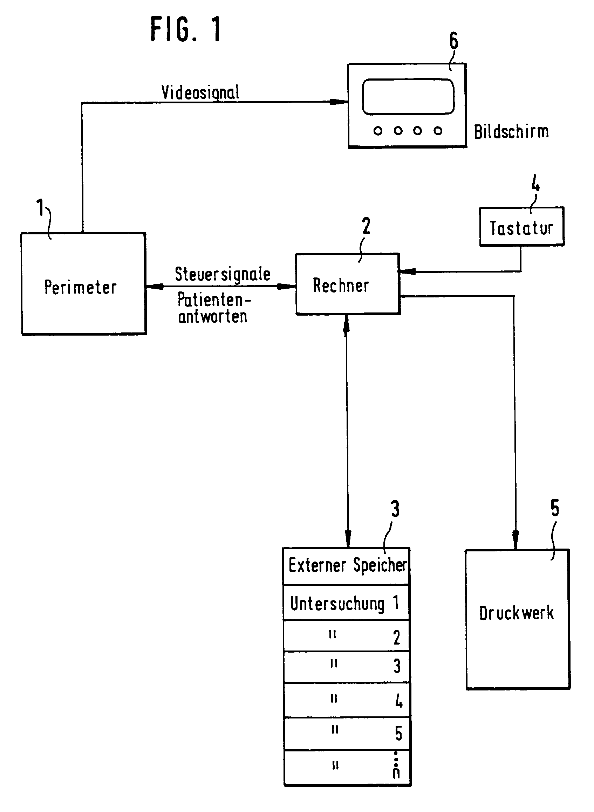

Das in Fig. 1 gezeigte automatische Perimeter weist das eigentliche Perimeter 1 auf. In diesem wird an einer bestimmten Beobachterstelle das zu untersuchende Patientenauge in einer Beobachtungsrichtung fixiert. Diesem werden mit geeigneten Mitteln in zeitlicher Staffelung die Stimuli im Umfeld der Beobachterachse präsentiert. Das eigentliche Perimeter 1 wird durch einen Rechner 2 mit internem Speicher gesteuert. Dem Rechner 2 ist wahlweise auch noch ein externer Speicher 3 zugeordnet. Um dem Rechner 2 Befehle zuzuleiten, ist ihm eine Tastatur 4 zugewiesen. Im internen Speicher des Rechners 2 und/oder im externen Speicher 3 werden von den verschiedenen Untersuchungen der Augen eines Patienten die ermittelten Schwellwerte der lokalen Lichtunterschiedsempfindlichkeit abgespeichert.

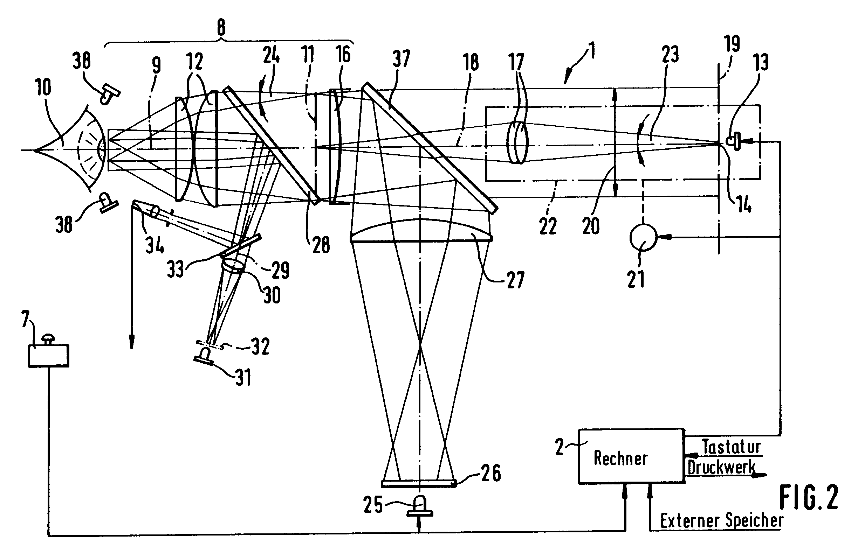

Mittels einem vom Rechner gesteuerten Druckwerk 5 können die in seinem internen oder externen Speicher 3 gespeicherten Untersuchungsresultate in aufbereiteter Form (numerisch oder graphisch) zur Darstellung gebracht werden. Der Operateur kann während der ganzen Untersuchung über einen Bildschirm 6 das Patientenauge beobachten und feststellen, ob dieses in Beobachtungsrichtung zentriert ist. Der Bildschirm 6 dient gleichzeitig der Kommunikation mit dem Rechner 2. Während einer Untersuchung antwortet jeweils der Patient indem er dem Rechner mittels einem Taster 7 (Fig.2) signalisiert ob er einen dargebotenen Stimulus gesehen hat oder nicht.

Wie Fig. 2 zeigt, weist das eigentliche Perimeter 1 ein Okular 8 auf, dessen optische Achse 9 mit der Beobachterachse eines an der Beobachterstelle 10 für eine Untersuchung fixierten Beobachterauges zusammenfällt. Die Linsen 12 des Okulars 8 sind auf eine Zwischenbildebene 11 (diese kann in Wirklichkeit leicht gekrümmt sein) fokusiert und derart geschliffen, dass sie die von einem Punkt der Zwischenbildebene 11 ausgehenden, divergierenden Strahlen parallel richten und gegen die Beobachtungsstelle 10 umlenken. Mit Bezug auf die Zwischenbildebene 11 bildet das Okular 8 eine Lupe. Mit dem parallelen Ausrichten der Strahlen durch die Linsen 12 gegen die Beobachterstelle 10 wird die Möglichkeit geschaffen, dass sich während einer Untersuchung das Beobachterauge in bestimmten Grenzen in einer Ebene senkrecht zur optischen Achse 9 verschieben darf, ohne dass deswegen ein dargebotener Stimulus unscharf (oder überhaupt nicht) wahrgenommen wird, oder dessen geometrische Lage vom Patienten verfälscht erkannt wird.

Weiter weist das eigentliche Perimeter 1 eine vom Rechner 2 gesteuerte Lichtquelle 13 vorzugsweise in Form einer Leuchtdiode auf. Die Verwendung einer Leuchtdiode hat den Vorteil, dass die Lichtquelle 13 verzögerungsfrei steuerbar ist. Zudem kann sie zur Durchführung der Farbperipherie leicht gegen eine farbige Leuchtdiode ausgetauscht werden. Es können indessen auch Halogenlampen oder Laserröhren als Lichtquelle verwendet werden.

Vom Licht der Lichtquelle 13 wird mittels einer Blende 14 (welche die Form und die Grösse eines Stimulus bestimmt) ein gebündelter Lichtstrahl ausgeblendet. Auf die Blende 14 ist ein Objektiv bestehend aus den Linsen 17 gerichtet, das von der Blende 14 bzw. einem Stimulus auf der Zwischenbildebene 11 ein reelles Zwischenbild erzeugt. Das Objektiv 17 ist mit seiner optischen Achse 18 parallel zu jener (9) des Okulars 8 orientiert. Das auf den Zwischenbildebene 11 erzeugte reelle Zwischenbild des Stimulus wird vom Beobachterauge durch das Okular 8 vergrössert wahrgenommen. Die Lichtquelle 13 und die Blende 14 sind gemeinsam in einer Ebene senkrecht zur optischen Achse 18 des Objektivs 17 verschiebbar. In der Ebene 19 können daher die Stimuli am vom Rechner 2 frei wählbaren, koordinatenmässig bestimmten Orten im vom Objektiv 17 erfassten Umfeld 20 dargeboten werden. Ein rechnergesteuerter Antrieb 21 dient der Lageverschiebung der Lichtquelle 13, der Blende 14 und des Objektivs 17, welche eine bauliche Einheit bilden und gemeinsam in einer Ebene senkrecht zur optischen Achse 18 verschiebbar sind. Vom Antrieb 21 wird jeweils die ganze Einheit 22 an den vom Rechner 2 befohlenen Ort bewegt. Durch die Beweglichkeit des Objektivs 17 ist dessen Durchmesser und damit die Grösse des Winkels 23 bei kleinem Abstand zur Blende 14 wählbar. Der Winkel 23 ist mindestens in grober Näherung, vorzugsweise gleich dem Winkel 24. Dessen Grösse bestimmt den Abstand von zwei von der Zwischenebene 11 ausgehenden, divergierenden und von der Doppellinse 12 parallel gerichteten Strahlen. Der Winkel 23 beträgt bei diesem Beispiel max. 20°; seine Grösse bestimmt die Abweichungstoleranz des Patientenauges von der Beobachterstelle 10.

Um für einen Patienten das ganze beobachtbare Umfeld der Beobachterachse 9 gleichmässig auszuleuchten, ist in der optischen Achse 18 ein gegen die Zwischenbildebene 11 gerichteter, teilweise lichtdurchlässiger Spiegel 37 angeordnet, der einerseits das durch das Objectiv 17 durchtretende Licht durchlässt und anderseits das von einer Lichtquelle 25 erzeugte Licht gegen die Zwischenbildebene umlenkt. Für eine gleichmässige Ausleuchtung des Umfeldes der Beobachterachse 9 sind zwischen der Lichtquelle 25 und dem Umlenkspiegel 24 eine Diffusorscheibe 26 und eine (z.B. als Fresnellinse gestaltete) Sammellinse 27 angeordnet. Die Diffusorscheibe kann für die Durchführung der Farbperimetrie zusätzlich als Farbfilter gestaltet sein.

Um einem Beobachterauge die Orientierung mit Bezug auf die Beobachterachse zu erleichtern, wird dem Patienten auf dieser Achse eine hell leuchtende Marke (Fixationsmarke) als Orientierungshilfe angeboten. Diesem Zweck dient ein zwischen der Zwischenebene 11 und der Doppellinse 12 angeordneter, teilweise lichtdurchlässiger, gegen die Beobachterstelle 10 gerichteter Umlenkspiegel 28. Den Schnittpunkt zwischen dem Umlenkspiegel 28 und der Beobachterachse 9 schneidet weiter die optische Achse 29 von zwei Linsen 30, die achsial mit einer Lichtquelle 31 und einer Blende 32 ausgerichtet ist. Die Blende 32 bestimmt Form und Grösse der Fixationsmarke.

Über einen weiteren in der optischen Achse 29 angeordneten Umlenkspiegel 33 (durchlässig für sichtbares Licht und reflektierend für infrarotes Licht) ist eine infrarot-empfindliche CCD-Kamera 34 auf die Beobachterachse 9 geschaltet, mit der ein Patientenauge während einer Untersuchung beobachtet werden kann. Die CCD-Kamera zeigt dem Operateur über den Monitor 6, ob das Patientenauge die für die Untersuchung richtige Lage einnimmt. Zu diesem Zwecke wird das Patientenauge 10 mit Infrarot-LED's 38 unsichtbar "beleuchtet".

Nach einem nicht dargestellten Ausführungsbeispiel können das Objektiv 17 (d.h. die Linsen 17) und die Feldlinsen 16 entfallen, wenn die Lichtquelle 13 und die Blende 14 soweit gegen das Okular verschoben werden, dass die Ebene 19 mit der Zwischenebene 11 zusammenfällt. Dies könnte beispielsweise mit einem Bildschirm in der Ebene 11 realisiert werden. Wird in der Zwischenebene 11 ein Bildschirm zur Darbietung des Stimulus angeordnet, besteht die Möglichkeit den Stimulus dunkler als das Umfeld zu wählen.

Bei beiden Ausführungsbeispielen wird von der Beobachterstelle 10 zur Zwischenebene 11 ein Abstand gewählt, der kleiner ist als die deutliche Sehweite.

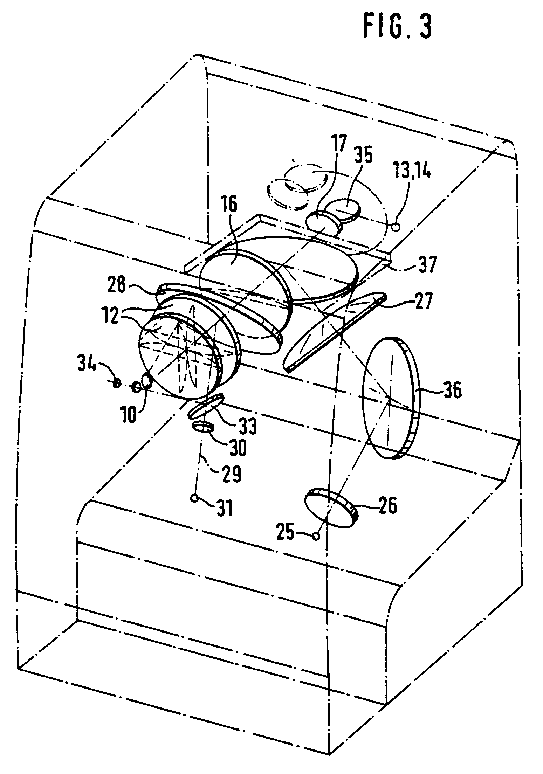

Die Fig. 3 zeigt das Beispiel nach Fig. 2 in einer räumlich kompakten Ausführungsform. Diese raumsparende Anordnung wird durch zwei Umlenkspiegel 35 und 36 erreicht, von denen der eine (35) zwischen der Blende 14 und den Linsen 17 und der andere (36) zwischen der Diffusorscheibe 26 und der Sammellinse 27 angeordnet ist. Bei dieser Ausführung kann das optische System zusammen mit dem Bildschirm 6 in einem vergleichsweise kleinen Gehäuse untergebracht werden. Es ist dem Fachmann naheliegend, durch das Einfügen weiterer Umlenkspiegel die ganze Vorrichtung noch gedrängter zu bauen.

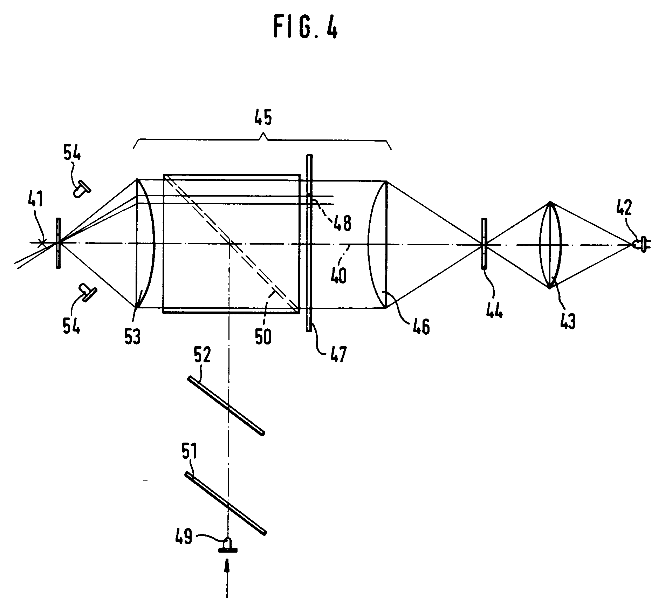

Im anhand Fig. 4 beschriebenen Beispiel basiert das optische System auf dem Prinzip der "Maxwellian View". Auf der Achse 40 des optischen Systems befindet sich einerseits die Beobachterstelle 41 und anderseits eine Lichtquelle 42 zur Erzeugung der Stimuli. Das Licht der Lichtquelle 42 wird durch eine Kondensorlinse 43 auf eine Blende 44 fokusiert und hinter der Blende 44 in einen parallelen Strahlengang 45 umgelenkt. Der Strahlengang 45 ist gegen die Blende 44 durch eine Feldlinse 46 und gegen die Beobachterstelle 41 durch eine Sammellinse 53 begrenzt. Die Beobachterstelle 41 ist so gestaltet, dass die Sammellinse 53 das aus dem Strahlengang 45 austretende Licht auf das Zentrum der Pupille des Beobachterauges fokusiert. Hierdurch bleiben Sehfehler des Beobachterauges ohne Einfluss auf die Stimulusabbildung auf der Netzhaut. Im parallelen Strahlengang 45 ist rechtwinklig zur Achse 40 eine den ganzen Querschnitt sperrende Blende 47 mit einer den Stimulus hinsichtlich Form und Grösse begrenzenden Blendenöffnung 48 angeordnet. Die Blende 47 ist nach zwei zueinander rechtwinklig orientierten Richtungen in einer Ebene senkrecht zur Achse 40 mittels eines nicht dargestellten und vom Rechner 2 gesteuerten Antriebs verschiebbar. Dadurch kann die Blendenöffnung 48 vom Rechner an jeden gewünschten und koordinatenmässig bestimmten Ort der Blendenebene verschoben werden. Die Darbietung der Stimuli erfolgt somit im parallelen Strahlengang 45 in der Blendenebene, von wo sie durch die Samellinse 53 auf der Netzhaut des Beobachterauges ausgebildet werden.

Die Umfeldbeleuchtung erfolgt von einer Streulichtquelle 49, deren diffuses Licht über einen teilweise lichtdurchlässigen Umlenkspiegel 50 im Strahlengang 45 gegen die Sammellinse 53 gelenkt wird. Über einen weiteren teildurchlässigen Umlenkspiegel 51 kann, wie beim ersten Ausführungsbeispiel, eine Fixationsmarke in das Umfeld eingeblendet werden, wobei ein weiterer Umlenkspiegel 52 (durchlässig für sichtbares Licht und reflektierend für infrarotes Licht) der Überwachung des Beobachterauges mit einer infrarot-empfindlichen Videokamera dient. Das Patientenauge wird dabei mittels der Infrarot-LED's 54 unsichtbar "beleuchtet".

In den beschreibenden Ausführungsbeispielen können sämtliche Linsen oder Linsengruppen durch ein ein- oder mehrlinsiges optisches System gebildet sein. Vorliegend werden - zur besseren Verständlichkeit - nur die einfachsten Ausführungsformen gezeigt und beschrieben.

## Patentansprüche

1. Vorrichtung zum Prüfen visueller Funktionen eines menschlichen Auges, mit einer Beobachterstelle (10, 41), an der ein zu untersuchendes Auge mit seiner optischen Achse in Richtung einer Beobachtungsachse (9, 40) zu fixieren ist, mit einer ersten Lichtquelle (13, 42) und dieser zugeordneten Mitteln (14 bzw. 43, 44, 46, 47, 48) zur zeitlich gestaffelten Erzeugung von Stimuli an wählbaren Orten im Umfeld der Beobachtungsachse (9, 40), wobei zwischen der Lichtquelle (13, 42) und der Beobachterstelle (10, 41) in der Beobachtungsachse (9, 40) ein optisches System angeordnet ist, welches auf der Netzhaut des an der Beobachterstelle (10, 41) fixierten Auges eine Abbildung der Stimuli erzeugt, dadurch gekennzeichnet, dass das optische System ein reelles Zwischenbild (11) der Stimuli erzeugt und der Beobachterstelle (10, 41) zugewandt, ein Okular (8, 53) für das fixierte Auge aufweist, das ihm ein vergrössertes, scharfes Abbild der Stimuli präsentiert und dass das Okular (8) eine, zwei oder mehrere Linsen (12) aufweist, welche die von einem Punkt des reellen Zwischenbildes ausgehenden, divergierenden Strahlen parallel gerichtet als Strahlenbündel gegen die Beobachterstelle (10) lenken, zum Zweck, dem fixierten Auge während einer Untersuchung eine dem Durchmesser des Strahlenbündels entsprechende Bewegungsfreiheit einzuräumen.

2. Vorrichtung nach Anspruch 1, dadurch gekennzeichnet, dass die wählbaren Orte, an denen die Stimuli erzeugt werden, wenigstens näherungsweise in einer Ebene (19) liegen.

3. Vorrichtung nach Anspruch 1 oder 2, dadurch gekennzeichnet, dass die Mittel zur Erzeugung der Stimuli Teil des optischen Systems sind und eine Blende (14) aufweisen, die vom Licht der ersten Lichtquelle (13) ein Lichtbündel ausblendet und die Form der Stimuli bestimmt.

4. Vorrichtung nach Anspruch 2 und 3, dadurch gekennzeichnet, dass zwischen der erwähnten Ebene (19) und dem Okular (8) ein sammelndes optisches System (17) angeordnet ist, das von den Stimuli in einer Ebene (11) das reelle Zwischenbild erzeugt, und dass das Okular (8) auf die Ebene (11) des reellen Zwischenbildes fokusiert ist.

5. Vorrichtung nach Anspruch 4, dadurch gekennzeichnet, dass der Scheitelwinkel (23) des von der Blende (14) ausgeblendeten Lichtbündels höchstens 20° beträgt.

6. Vorrichtung nach einem der Ansprüche 1 bis 4, gekennzeichnet durch eine zweite Lichtquelle (25) aufweisende Umfeldbeleuchtungsmittel (25, 26, 27, 37) zur Erzeugung von im Umfeld der Beobachtungsachse (9) gleichmässig verteiltem, diffusem Licht, das hinsichtlich zeitlicher Darbietung, Amplitude und/oder Wellenlänge von jenem der Stimuli verschieden ist.

7. Vorrichtung nach einem der Ansprüche 1 bis 6, dadurch gekennzeichnet, dass den Mitteln (14) zur Erzeugung der Stimuli und/oder den Umfeldbeleuchtungsmitteln (25, 26, 27, 37) Elemente zur Wahl des Leuchtdichteprofils zugeordnet sind.

8. Vorrichtung nach einem der Ansprüche 1 bis 7, dadurch gekennzeichnet, dass der ersten Lichtquelle (13) zur Erzeugung der Stimuli Einstellorgane zur Wahl des zeitlichen Verlaufs der Amplitude und/oder der Wellenlänge der Stimuli zugeordnet sind.

9. Vorrichtung nach Anspruch 4, dadurch gekennzeichnet, dass die erste Lichtquelle (13) und die Blende (14) nach zwei sich auf einer Ebene senkrecht zur optischen Achse (18) des sammelnden optischen Systems (17) rechtwinklig schneidenden Richtungen verschiebbar sind.

10. Vorrichtung nach Anspruch 9, dadurch gekennzeichnet, dass das der Blende (14) zugewandte, sammelnde optische System (17) mit dieser zusammen verschiebbar ist.

11. Vorrichtung nach Anspruch 1, dadurch gekennzeichnet, dass zwischen dem Okular (8) und der Beobachterstelle (10) eine Halterung für Korrekturgläser zum Ausgleich von sphärischen und zylindrischen Sehfehlern des zu untersuchenden Auges vorhanden ist.

12. Vorrichtung nach Anspruch 1, dadurch gekennzeichnet, dass mindestens eine Linse (12) des Okulars (8) in Richtung seiner optischen Achse (9) zur Korrektur von sphärischen Sehfehlern verstellbar ist.

13. Vorrichtung nach einem der Ansprüche 1 bis 12, dadurch gekennzeichnet, dass in der optischen Achse (9) des optischen Systems ein teilweise lichtdurchlässiger erster Umlenkspiegel (28) angeordnet ist und dass eine dritte, einen gebündelten Lichtstrahl erzeugende Lichtquelle (31) mittels einer Linse (30) gegen die Schnittstelle zwischen der optischen Achse (9) und dem ersten Umlenkspiegel (28) gerichtet ist, um auf der Beobachtungsachse (9) eine Fixationsmarke zu erzeugen.

14. Vorrichtung nach Anspruch 13, dadurch gekennzeichnet, dass in der optischen Achse (29) der erwähnten Linse (30) ein zweiter, im sichtbaren Bereich lichtdurchlässiger und im infraroten Bereich reflektierender und gegen den ersten Umlenkspiegel (28) gerichteter Umlenkspiegel (33) vorhanden ist, und dass eine mit einem Monitor (6) verbundene infrarot-empfindliche Videokamera (34) auf die Schnittstelle zwischen der optischen Achse der Linse (30) und dem zweiten Umlenkspiegel (33) gerichtet ist.

15. Vorrichtung nach Anspruch 14, dadurch gekennzeichnet, dass die Beobachtungsstelle (10) mit Infrarotlichtquellen beleuchtbar und die Videokamera (34) eine infrarot-empfindliche CCD-Kamera ist.

16. Vorrichtung nach Anspruch 15, dadurch gekennzeichnet, dass in der optischen Achse (18) des optischen Systems ein dritter teilweise lichtdurchlässiger Umlenkspiegel (37) vorhanden ist, der das Licht der zweiten Lichtquelle (25) gegen die Beobachterstelle (10) umlenkt.

17. Vorrichtung nach Anspruch 16, dadurch gekennzeichnet, dass der dritte Umlenkspiegel (37) in der optischen Achse (9) des Okulars (8) liegt.

18. Vorrichtung nach Anspruch 16 oder 17, dadurch gekennzeichnet, dass zwischen der zweiten Lichtquelle (25) und dem dritten Umlenkspiegel (37) eine Diffusorscheibe (26) sowie eine gegen den dritten Umlenkspiegel (37) gerichtete Sammellinse (27) vorhanden ist.

19. Vorrichtung nach Anspruch 18, dadurch gekennzeichnet, dass im Strahlengang der zweiten Lichtquelle (25) zwischen der Diffusorscheibe (26) und der Sammellinse (27) eine Umlenkung mittels eines vierten Umlenkspiegels (36) vorgesehen ist.

20. Vorrichtung nach Anspruch 4 oder 10, dadurch gekennzeichnet, dass zwischen der Blende (14) und dem ihr zugewandten sammelnden optischen System (17) ein fünfter Umlenkspiegel (35) angeordnet ist.

21. Vorrichtung nach Anspruch 2, dadurch gekennzeichnet, dass die Ebene durch die Bildscheibe eines Monitors gebildet wird und der Monitor die Stimuli auf seiner Bildscheibe erzeugt.

## Claims

1. An apparatus for examining the visual functions of a human eye, with an observer location (10, 41) at which the eye to be examined is to be fixed with its optical axis in the direction of an observation axis (9, 40), with means (14; 43, 44, 46, 47, 48), associated with a first light source (13,42), for the temporally staggered production of stimuli at selectable locations in the surrounding field of the observation axis (9, 40), wherein an optical system is disposed on the observation axis (9, 40) between the light source (13, 42) and the observer location (10, 41), which optical system produces an image of the stimuli on the retina of the eye fixed at the observer location (10, 41), characterised in that the optical system produces a real first image (11) of the stimuli, comprises an eyepiece (8, 53) for the fixed eye and facing the observer location (10, 41), and presents the eye with an enlarged, sharp image of the stimuli, that the eyepiece (8) comprises one, two or more lenses (12) which direct the diverging rays coming from a point on the real first image towards the observer location (10) as a beam with parallel orientation, for the purpose of allowing the fixed eye a freedom of movement corresponding to the diameter of the beam during an examination.

2. An apparatus according to claim 1, characterised in that the selectable locations at which the stimuli are produced lie at least approximately in one plane (19).

3. An apparatus according to claim 1 or 2, characterised in that the means for producing the stimuli are part of the optical system and comprise a diaphragm (14) which extracts a light beam from the light of the first light source (13) and determines the form of the stimuli.

4. An apparatus according to claims 2 and 3, characterised in that a convergent optical system (17), which produces the real first image in one plane (11) from the stimuli, is disposed between the said plane (19) and the eyepiece (8), and that the eyepiece (8) is focused on the plane (11) of the real first image.

5. An apparatus according to claim 4, characterised in that the vertex angle (23) of the light beam extracted by the diaphragm (14) is at most 20°.

6. An apparatus according to any one of claims 1 to 4, characterised by surrounding field illumination means (25, 26, 27, 37) having a second light source (25) for producing uniformly distributed diffuse light, which differs from each of the stimuli in terms of temporal presentation, amplitude and/or wavelength, in the surrounding field of the observation axis (9).

7. An apparatus according to any one of claims 1 to 6, characterised in that elements for selecting the luminance profile are associated with the means (14) for producing the stimuli and/or with the surrounding field illumination means (25, 26, 27, 37).

8. An apparatus according to any one of claims 1 to 7, characterised in that adjustment elements for selecting the temporal progression of the amplitude and/or the wavelength of the stimuli are associated with the first light source (13) for producing the stimuli.

9. An apparatus according to claim 4, characterised in that the first light source (13) and the diaphragm (14) are displaceable in two directions which intersect each other at right angles on a plane perpendicular to the optical axis (18) of the convergent optical system (17).

10. An apparatus according to claim 9, characterised in that the convergent optical system (17) facing the diaphragm (14) is displaceable together with the latter.

11. An apparatus according to claim 1, characterised in that a holder for correcting glasses for compensating for spherical and cylindrical visual defects in the eye to be examined is present between the eyepiece (8) and the observer location (10).

12. An apparatus according to claim 1, characterised in that at least one lens (12) of the eyepiece (8) can be adjusted in the direction of its optical axis (9) for the correction of spherical visual defects.

13. An apparatus according to any one of claims 1 to 12, characterised in that a partially transparent first tilted mirror (28) is disposed on the optical axis (9) of the optical system and that a third light source (31), which produces a directional light beam, is oriented by means of a lens (30) towards the point of intersection between the optical axis (9) and the first tilted mirror (28), in order to produce a fixation mark on the observation axis (9).

14. An apparatus according to claim 13, characterised in that a second tilted mirror (33), which is transparent in the visible region and reflecting in the infrared region and which is oriented towards the first tilted mirror (28), is present on the optical axis (29) of the said lens (30), and that an infrared-sensitive video camera (34) connected to a monitor (6) is set on the point of intersection between the optical axis of the lens (30) and the second tilted mirror (33).

15. An apparatus according to claim 14, characterised in that the observation location (10) can be illuminated by means of infrared light sources and the video camera (34) is an infrared-sensitive CCD camera.

16. An apparatus according to claim 15, characterised in that a third partially transparent tilted mirror (37), which deflects the light from the second light source (25) towards the observer location (10), is present on the optical axis (18) of the optical system.

17. An apparatus according to claim 16, characterised in that the third tilted mirror (37) is situated on the optical axis (9) of the eyepiece (8).

18. An apparatus according to claim 16 or 17, characterised in that a diffuser disc (26), and a convergent lens (27) oriented towards the third tilted mirror (37), are present between the second light source (25) and the third tilted mirror (37).

19. An apparatus according to claim 18, characterised in that a deflection is provided between the diffuser disc (26) and the convergent lens (27) in the beam path of the second light source (25) by means of a fourth tilted mirror (36).

20. An apparatus according to claim 4 or 10, characterised in that a fifth tilted mirror (35) is disposed between the diaphragm (14) and the convergent optical system (17) facing it.

21. An apparatus according to claim 2, characterised in that the plane is formed by the screen of a monitor and the monitor produces the stimuli on its screen.

## Revendications

1. Dispositif pour contrôler les fonctions visuelles d'un oeil humain, comprenant un poste d'observation (10, 41) auquel un oeil à contrôler est maintenu de manière que son axe optique soit en direction d'un axe d'observation (10,40), comprenant une première source de lumière (13, 42) et des moyens (14 ou 43, 44, 46, 47, 48) associés à cette dernière pour produire de façon étagée dans le temps des stimuli en des emplacements sélectionnables du champ périphérique de l'axe d'observation (9, 40), un système optique étant monté entre la source de lumière (13, 42) et le poste d'observation (10, 41) dans l'axe d'observation (9, 40), système qui produit sur la rétine de l'oeil maintenu au poste d'observation (10, 41) une image des stimuli, caractérisé en ce que le système optique produit une image intermédiaire réelle (11) des stimuli et comprend un oculaire (8, 53) tourné vers le poste d'observation (10, 41) de l'oeil qui y est maintenu, qui présente une image des stimuli agrandie et plus précise, et en ce que l'oculaire (8) comprend une ou plusieurs lentilles (12) qui renvoient les rayons divergents provenant d'un point de l'image intermédiaire réelle en les dirigeant parallèlement sous forme d'un faisceau vers le poste d'observation (10) afin de permettre à l'oeil maintenu de façon fixe pendant un examen une liberté de mouvement correspondant au diamètre du faisceau.

2. Dispositif selon la revendication 1, caractérisé en ce que les emplacements sélectionnables où sont produits les stimuli sont situés au moins approximativement dans un plan (19).

3. Dispositif selon la revendication 1 ou 2, caractérisé en ce que les moyens pour produire les stimuli constituent une partie du système optique et comprennent un diaphragme (14) qui délimite un faisceau lumineux de la lumière de la première source de lumière (13) et détermine la forme des stimuli.

4. Procédé selon les revendications 2 et 3, caractérisé en ce qu'entre le plan mentionné (19) et l'oculaire (8) est disposé un système optique convergent (17) qui produit l'image intermédiaire réelle des stimuli dans un plan (11) et en ce que l'oculaire (8) est focalisé sur le plan (11) de l'image intermédiaire réelle.

5. Procédé selon la revendication 4, caractérisé en ce que l'angle au sommet (23) du faisceau lumineux délimité par le diaphragme (14) est au maximum de 20°.

6. Dispositif selon l'une quelconque des revendications 1 à 4, caractérisé par des moyens d'éclairage de champ périphérique (25, 26, 27, 37) comprenant une seconde source de lumière (25) pour produire une lumière diffuse répartie régulièrement dans le champ périphérique de l'axe d'observation (9) et qui est différente de celle des stimuli en ce qui concerne sa présentation dans le temps, son amplitude et/ou sa longueur d'onde.

7. Dispositif selon l'une quelconque des revendications 1 à 6, caractérisé en ce que des éléments de sélection du profil de luminance sont subordonnés au moyen (14) pour produire les stimuli et/ou aux moyens d'éclairage de champ périphérique (25, 26, 27, 37).

8. Dispositif selon l'une quelconque des revendications 1 à 7, caractérisé en ce que des organes de commande pour sélectionner l'évolution dans le temps de l'amplitude et/ou de la longueur d'onde des stimuli sont associés à la première source de lumière (13).

9. Dispositif selon la revendication 4, caractérisé en ce que la première source de lumière (13) et le diaphragme (14) peuvent être déplacés dans deux directions se coupant à angle droit dans un plan perpendiculaire à l'axe optique (18) du système optique convergent (17).

10. Dispositif selon la revendication 9, caractérisé en ce que le système optique convergent (17) qui est tourné vers le diaphragme (14) peut être déplacé en commun avec lui.

11. Dispositif selon la revendication 1, caractérisé en ce qu'une monture pour des verres correcteurs permettant de compenser des défauts de vision sphériques et cylindriques de l'oeil à contrôler sont prévus entre l'oculaire (8) et le poste d'observation (10).

12. Dispositif selon la revendication 1, caractérisé en ce qu'au moins une lentille (12) de l'oculaire (8) peut être déplacée dans la direction de son axe optique (9) pour corriger des défauts de vision sphériques.

13. Dispositif selon l'une quelconque des revendications 1 à 12, caractérisé en ce qu'un premier miroir de renvoi partiellement transparent (28) est disposé dans l'axe optique (9) du système optique et en ce qu'une troisième source de lumière (31) qui produit un rayon lumineux rassemblé en faisceau est orientée au moyen d'une lentille (30) vers la position d'intersection entre l'axe optique (9) et le premier miroir de renvoi (28) pour produire une marque de fixation sur l'axe d'observation (9).

14. Dispositif selon la revendication 13, caractérisé en ce qu'un second miroir de renvoi (33) dirigé vers le premier miroir de renvoi (28), transparent dans la plage visible et réfléchissant dans la plage des infrarouges, est prévu dans l'axe optique (29) de ladite lentille (30), et en ce qu'une caméra vidéo (34) sensible aux infrarouges et reliée à un moniteur (6) est orientée sur la position d'intersection entre l'axe optique de la lentille (30) et le second miroir de renvoi (33).

15. Dispositif selon la revendication 14, caractérisé en ce que le poste d'observation (10) peut être éclairé par des sources de lumière infrarouge et la caméra vidéo (34) est une caméra CCD sensible aux infrarouges.

16. Dispositif selon la revendication 15, caractérisé en ce qu'il est prévu un troisième miroir de renvoi partiellement transparent (37) dans l'axe optique (18) du système optique, qui renvoie la lumière de la seconde source de lumière (25) vers le poste d'observation (10).

17. Dispositif selon la revendication 16, caractérisé en ce que le troisième miroir de renvoi (37) est disposé dans l'axe optique (9) de l'oculaire (8).

18. Dispositif selon la revendication 16 ou 17, caractérisé en ce qu'un disque diffuseur (26) ainsi qu'une lentille convergente (27) orientée vers le troisième miroir de renvoi (37) sont prévus entre la seconde source de lumière (25) et le troisième miroir de renvoi (37).

19. Dispositif selon la revendication 18, caractérisé en ce qu'il est prévu sur le parcours du faisceau de la seconde source de lumière (25) un renvoi au moyen d'un quatrième miroir de renvoi (36) entre le disque diffuseur (26) et la lentille convergente (27).

20. Dispositif selon la revendication 4 ou 10, caractérisé en ce qu'un cinquième miroir de renvoi (35) est monté entre le diaphragme (34) et le système optique convergent (17) tourné vers lui.

21. Dispositif selon la revendication 2, caractérisé en ce que le plan est formé par l'écran d'un moniteur et le moniteur produit les stimuli sur son écran.
